# EUROPEAN PATENT APPLICATION

(11) **EP 4 266 074 A1**
(43) Date of publication of application: **25.10.2023**
(21) Application number: 22169368.2
(22) Date of filing: 22.04.2022
(51) Int. Cl.: G01R 33/56, G01R 33/561, G06N 3/04, G06T 7/00, G01R 33/48, G01R 33/50

(54) **SEGMENTATION OF MEDICAL IMAGES RECONSTRUCTED FROM A SET OF MAGNETIC RESONANCE IMAGES**

(71) Applicant: Koninklijke Philips N.V., 5656 AG Eindhoven (NL)
(72) Inventor: VAN DEN BRINK, Johan Samuel, Eindhoven (NL)
(74) Representative: Philips Intellectual Property & Standards

(57) **Abstract**

Disclosed herein is a medical system (100, 300) comprising a memory (110) storing machine executable instructions (120) and an image processing convolutional neural network (122). The image processing convolutional neural network is configured to output a segmentation (128) of a field of view (309) of a subject (318) in response to inputting a predetermined set of magnetic resonance images (124) into the image processing convolutional neural network. The medical system further comprises a computational system (104). Execution of the machine executable instructions causes the computational system to: receive (200) the predetermined set of magnetic resonance images; reconstruct (202) a combined medical image (126) of the field of view from the predetermined set of magnetic resonance images; and receive (204) the segmentation in response to inputting the predetermined set of magnetic resonance images into the image processing convolutional neural network. The combined medical image is a non-linear combination of the magnetic resonance images.

## Description

### FIELD OF THE INVENTION

The invention relates to magnetic resonance imaging, in particular to medical images constructed from magnetic resonance images.

### BACKGROUND

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially and imaged using MRI. A difficulty with performing magnetic resonance imaging is that it takes time to acquire the k-space data necessary to reconstruct the magnetic resonance imaging. When the k-space data is acquired different imaging protocols can be used to weight the image differently. For example, a magnetic resonance image could be weighted to show proton density, the spatial variation of T1, or the spatial variation of T2 or T2-star. These different weightings are commonly referred to as "contrasts" or "contrast types." Physicians and other medical professionals are accustomed to viewing particular "contrasts." Other imaging techniques have been developed in recent years such as magnetic resonance imaging fingerprinting (MRF) or synthetic MR. In these techniques, k-space data is used to acquire images with atypical contrast weightings, which may be used to construct synthetic or mapped images that mimic or reproduce typical or canonical "contrasts."

United State patent application publication US2021174937 (A1) discloses a raw diagnostic machine for a medical diagnosis of raw medical imaging data generated by a medical imaging machine as opposed to a medical diagnosis of a medical image conventionally reconstructed from the raw medical imaging data. In operation, the raw diagnostic engine includes a medical imaging diagnostic controller implementing a dimension reduction pre-processor for selecting or extracting one or more dimension reduced feature vectors from the raw medical imaging data, and further implementing a raw diagnostic artificial intelligence engine for rendering a diagnostic assessment of the raw medical imaging data as represented by the dimension reduced feature vector(s). The medical imaging diagnostic controller may further control a communication of the diagnostic assessment of the raw medical imaging data (e.g., a display, a printing, an emailing, a texting, etc.).

### SUMMARY OF THE INVENTION

The invention provides for a medical system, a computer program, and a method in the independent claims. Embodiments are given in the dependent claims.

A difficulty in using a combined medical image which is reconstructed from magnetic resonance images is that although it appears to a human to be an authentic magnetic resonance or other type of medical image, the resulting image can have fundamental differences from a real image. For example, if a predetermined set of magnetic resonance images is used to reconstruct a combined medical image that is a synthetic T1 weighted magnetic resonance image it is possible that automatic segmentation algorithms could fail. This is particularly true if the segmentation is implemented using a neural network. One reason for this is that the background noise is different from an image reconstructed directly from k-space data.

Embodiments may provide a means for accurately and consistently segment a combined medical image by segmenting the set of predetermined magnetic resonance images instead of segmenting the combined medical image. The set of predetermined magnetic resonance images are magnetic resonance images, so they have a natural distribution on noise. This may provide a means of consistently and accurately segmenting the combined medical image.

In one aspect the invention provides for a medical system that comprises a memory that stores both machine-executable instructions and an image processing convolutional neural network. An image processing convolutional neural network is a convolutional neural network that is configured to receive one or more images as input and to output one or more images. The image processing convolutional neural network is configured to output a segmentation of a field of view of a subject in response to inputting a predetermined set of magnetic resonance images that are according to a magnetic resonance imaging protocol into the image processing convolutional neural network.

Examples of architectures which are suitable for implementing the image processing convolutional neural network are the ResNet and U-Net neural network architectures, and extensions thereof using generative adaptive networks (GAN). The image processing neural network can be trained by collecting predetermined sets of magnetic resonance images and manually segmenting the resulting combined medical image. A predetermined set of magnetic resonance images can be input into the untrained or partially trained image processing convolutional neural network and its output can be compared to the manual segmentation to perform, for example, deep learning.

Each of the predetermined set of magnetic resonance images is descriptive of the field of view of the subject. The medical system further comprises a computational system.

Execution of the machine-executable instructions causes the computational system to receive the predetermined set of magnetic resonance images. Execution of the machine-executable instructions further causes the computational system to reconstruct a combined medical image of the field of view from the predetermined set of magnetic resonance images. The combined medical image is a non-linear combination of the magnetic resonance images. In other words, the combined medical image is reconstructed from the predetermined set of magnetic resonance images using a non-linear algorithm. Examples include reconstructing the image using a neural network or performing a non-liner optimization to reconstruct an image using magnetic resonance fingerprinting.

Execution of the machine-executable instructions further causes the computational system to receive the segmentation in response to inputting the predetermined set of magnetic resonance images into the image processing convolutional neural network.

In this embodiment the predetermined set of magnetic resonance images is used to reconstruct the combined medical image. Because the combined medical image is not identical with one of the set of magnetic resonance images it may have various image properties or processes which may prohibit or make it more difficult to derive proper segmentations. Determining the segmentation directly from the predetermined set of magnetic resonance images may therefore enable a more accurate segmentation. Typically, segmentation algorithms or neural networks trained for doing segmentation, rely heavily on basic image properties such as the noise spectrum or power spectrum of noise within the image.

The combined medical image however, may differ from what would be expected by one of these algorithms or convolutional neural networks within the segmentation. The use of the image processing convolutional neural network to do the segmentation directly on the predetermined set of magnetic resonance images may therefore provide for a means of consistently and accurately segmenting the field of view. Since the combined medical image depicts the field of view the segmentation may be used as the segmentation of the combined medical image.

In another embodiment the machine-executable instructions are configured such that the computational system is able to reconstruct the combined medical image when the predetermined set of magnetic resonance images have or has a predetermined distribution of magnetic resonance image weightings or magnetic resonance imaging contrasts or contrast types. For example, the magnetic resonance imaging protocol may specify the acquisition parameters or modify the pulse sequence used to acquire the predetermined set of magnetic resonance images such that they have a predetermined distribution of contrasts or image weightings.

This may enable the reconstruction of different types of combined medical images. For example, the combined medical image could be a medical image that is reconstructed using magnetic resonance fingerprinting. In other examples, the combined medical image could be a synthetically generated T1 or T2 weighted image. Such a synthetically created T1 or T2 image would have the appearance of a T1 or T2 image to a human but the noise profile would likely prevent a neural network from performing a segmentation properly.

In another embodiment the segmentation is a segmentation of the combined medical image. This may be true because the combined medical image depicts the field of view.

In another embodiment each of the predetermined set of magnetic resonance images is a non-clinical magnetic resonance image. A non-clinical magnetic resonance image as used herein encompasses a magnetic resonance image which does not have a clear weighting profile. For example, the non-clinical magnetic resonance image may not be configured in a way which a human could interpret the image properly. In another embodiment each of the predetermined set of magnetic resonance images is a non-canonical (or atypical) magnetic resonance image. As used herein, a non-canonical magnetic resonance image is a magnetic resonance imaging with a contrast or weighting type which is different from what may be used for clinical use.

In another embodiment the memory further comprises an image reconstruction neural network configured to output the combined medical image in response to receiving the predetermined set of magnetic resonance images as an input. The combined medical image is reconstructed by inputting the predetermined set of magnetic resonance images into the image reconstruction neural network. In this embodiment, the image processing convolutional neural network which does the segmentation is separate from the image reconstruction neural network. This embodiment may be beneficial because it may provide for an independent and accurate means of providing the segmentation.

The image reconstruction neural network can for example be implemented using a ResNet or U-net neural network, or with extensions thereof using GANs. The training data can for example be collected by using an algorithm to reconstruct the combined image from the predetermined set of magnetic resonance image. The training data can then be used to train the image reconstruction neural network.

In another embodiment the image processing convolutional neural network is further configured for outputting the combined medical image in response to receiving the predetermined set of magnetic resonance images as input. The combined medical image is reconstructed by inputting the predetermined set of magnetic resonance images into the image processing convolutional neural network. This embodiment may be beneficial because the same neural network is used for both the segmentation and the image reconstruction.

In another embodiment the image processing convolutional neural network comprises a single encoder branch configured for receiving the predetermined set of magnetic resonance images as input. The image processing convolutional neural network further comprises a first decoder branch and a second decoder branch each connected to the single encoder branch. The first decoder branch is configured to output the combined medical image. The second decoder branch is configured to output the segmentation of the combined medical image. This architecture may be similar to what is known in some research papers as a Y-Net. This may be equivalent to a U-Net that has multiple decoder branches.

The architecture of the Y-net may, for example, be taken from a U-net where a second encoding branch is added. The Y-net may be trained by taking a predetermined set of magnetic resonance images and reconstructing a training combined image using an algorithm. A training segmentation may then be performed on the training combined image manually. The training data may then be provided by paring the predetermined set of magnetic resonance images with the training combined image and the training segmentation.

The image processing neural network may have more than a first and second decoder branch. There could for example, be additional branches that are trained for performing specific segmentation or image processing tasks.

In another embodiment the image processing convolutional neural network further comprises multiple skip connections between the first decoder branch and the second decoder branch. This embodiment may be beneficial because it may ensure that the segmentation and the combined magnetic resonance image more accurately match each other.

In another embodiment the machine-executable instructions are further configured to reconstruct the combined medical image algorithmically. The combined medical image for example may be a medical image that is reconstructed with a well-known algorithm such as is used for magnetic resonance fingerprinting.

In another embodiment, the magnetic resonance imaging protocol is any one of the following: a magnetic resonance fingerprinting protocol, a SyntAc protocol, a QRAPMASTER protocol, a QALAS protocol, a MaGIC protocol, a MP2RAGEME protocol, a EPImix protocol, a DESS magnetic resonance imaging protocol, a TESS magnetic resonance imaging protocol, an ultrashort echo time magnetic resonance imaging protocol, a DIXON magnetic resonance imaging protocol, a SAGE magnetic resonance imaging protocol, a QuICS magnetic resonance imaging protocol, and an MRSTAT magnetic resonance imaging protocol.

SyntAc is a 2D or 3D imaging method that uses Cartesian k-space sampling. It may be referred to as QRAPMASTER (2D), QALAS or MaGIC (from GE). These gradient echo sequences provide a set of data with multiple T2 weightings (T2 preparation pulses) and T1 weightings (inversion times).

MP2RAGEME is an imaging protocol that provides T1, T2^{∗}, and QSM mapping using single echo first inversion and second inversion multi-echo. It uses "Standard" TSE images, but those "standard" images aren't representative of radiological guidelines/needs (what I called "non-radiological")

An extension of this method would, of course, be to use 2-echo (DESS) or 3-echo (TESS) readouts as the magnetic resonance imaging protocol used to acquire the k-space data. DESS and TESS are also well-known examples where software normally generates a weighted maximum intensity projection (wMIP) from the two or three input images. The sources images may be better when exploiting NNs for analysis or segmentation.

Ultra-short echo time (UTE) may be used as the predetermined set of magnetic resonance images, because in UTE, at least 2 echo times are acquired (ultrashort and short).

Examples are also applicable to DIXON magnetic resonance imaging. In DIXON imaging, images are typically acquired for 2 to 5 echo times.

SAGE also produces a set off predetermined magnetic resonance images. Multi-echo gradient echo imaging is performed for 5 to 7 echo times using T2-star weighting, plus inversion recovery steps.

MR Multitasking, includes diffusion weighted imaging. The multiple images used for the diffusion weighted images may be used as the predetermined set off magnetic resonance images.

In another embodiment wherein the combined medical image is any one of the following: a synthetic magnetic resonance image, a synthetic proton density weighted magnetic resonance image, a synthetic T1 weighted magnetic resonance image, a synthetic T2 weighted magnetic resonance image, a synthetic T2-star weighted magnetic resonance image, a synthetic FLAIR magnetic resonance image, a quantitative map, a pseudo computed tomography image, and a pseudo-X-ray image.

In another embodiment the medical system further comprises a magnetic resonance imaging system. The memory further contains pulse sequence commands configured to control the magnetic resonance imaging system to acquire k-space data according to the magnetic resonance imaging protocol. Execution of the machine-executable instructions further causes the computational system to acquire the k-space data by controlling the magnetic resonance imaging system and to reconstruct the predetermined set of magnetic resonance images from the k-space data.

In another embodiment the segmentation of the combined medical image comprises any one of the following: an anatomical segmentation, identification of a damaged tissue region, an identification of a likely anatomical abnormality region, and identification of a likely lesion region. These embodiments may be beneficial because they may not only provide segmentation, but they may also provide for a more accurate identification for assisting physicians in identifying damage or diseased portions.

In another aspect the invention provides for a computer program or a computer program product that comprises machine-executable instructions and an image processing convolutional neural network. The image processing convolutional neural network is also executable code or instructions. The image processing convolutional neural network is configured to output a segmentation of a field of view of a subject in response to inputting a predetermined set of magnetic resonance images that depict the subject in the field of view according to a magnetic resonance imaging protocol into the image processing convolutional neural network. Each of the predetermined set of magnetic resonance images is descriptive of the field of view of the subject. Execution of the machine-executable instructions causes the computational system to receive the predetermined set of magnetic resonance images. Execution of the machine-executable instructions further causes the computational system to reconstruct a combined medical image of the field of view from the predetermined set of magnetic resonance images. The combined medical image is a non-linear combination of the magnetic resonance images. Execution of the machine-executable instructions further causes the computational system to receive the segmentation in response to inputting the predetermined set of magnetic resonance images into the image processing convolutional neural network.

In another aspect the invention provides for a method of medical imaging. The method comprises receiving a predetermined set of magnetic resonance images. Each of the predetermined set of magnetic resonance images is descriptive of a field of view of the subject. The method further comprises reconstructing a combined medical image of the field of view from the predetermined set of magnetic resonance images. The combined medical image is a non-linear combination of the magnetic resonance images. The method further comprises receiving a segmentation in response to inputting the predetermined set of magnetic resonance images into an image processing convolutional neural network. The image processing convolutional neural network is configured to output the segmentation of the field of view of the subject in response to inputting a predetermined set of magnetic resonance images according to a magnetic resonance imaging protocol into the image processing convolutional neural network.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image or MR image is defined herein as being the reconstructed two- or three-dimensional visualization of anatomic data contained within the k-space data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates an example of a medical system;
Fig. 4 shows a flow chart which illustrates a method of using the medical system of Fig. 3;
Fig. 5 Illustrates a means of generating the segmentation and the combined medical image;
Fig. 6 Illustrates a further means of generating the segmentation and the combined medical image;
Fig. 7 Illustrates a further means of generating the segmentation and the combined medical image;
Fig. 8 Illustrates a further means of generating the segmentation and the combined medical image;
Fig. 9 illustrates an example of a predetermined set of magnetic resonance images; and
Fig. 10 illustrates an example of several different combined medical images.

### DESCRIPTION OF EMBODIMENTS

Like numbered elements in these figures are either equivalent elements or perform the same function. Elements which have been discussed previously will not necessarily be discussed in later figures if the function is equivalent.

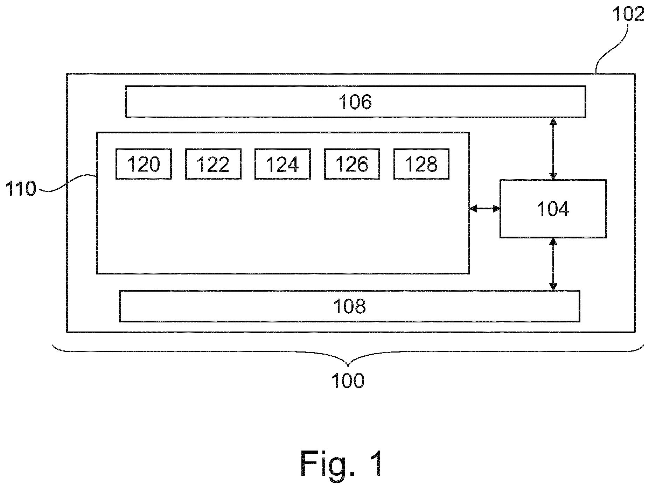

Fig. 1 illustrates an example of a medical system 100. In this example the medical system 100 comprises a computer 102 with a computational system 104. The computer 102 is intended to represent one or more computers or computer systems. The computational system 104 is intended to represent one or more computational systems or computational cores. The computer 102 is further shown as containing an optional hardware interface 106 which is connected to the computational system 104. If other components of the medical system 100 are present or included such as a magnetic resonance imaging system, then the hardware interface 106 could be used to exchange data and commands with these other components. The medical system 100 is further shown as comprising an optional user interface 108 which may provide various means for relaying data and receiving data and commands from an operator.

The medical system 100 is further shown as comprising a memory 110 that is connected to the computational system 104. The memory 110 is intended to represent various types of memory which may be accessible to the computational system 104. The memory 110 is shown as containing machine-executable instructions 120. The machine-executable instructions 120 are instructions which enable the computational system 104 to perform various control, data processing, and image processing tasks. The memory 110 is further shown as containing the image processing convolutional neural network 122. The memory 110 is further shown as containing a predetermined set of magnetic resonance images 124. The image processing convolutional neural network 122 is configured for receiving the predetermined set of magnetic resonance images 124 and in response outputting a segmentation of a field of view. The memory 110 is further shown as containing a combined medical image 126 that was reconstructed either algorithmically or via another neural network from the predetermined set of magnetic resonance images 124. And finally, the memory 110 is shown as comprising a segmentation 128 that was obtained by inputting the predetermined set of magnetic resonance images 124 into the image processing convolutional neural network 122.

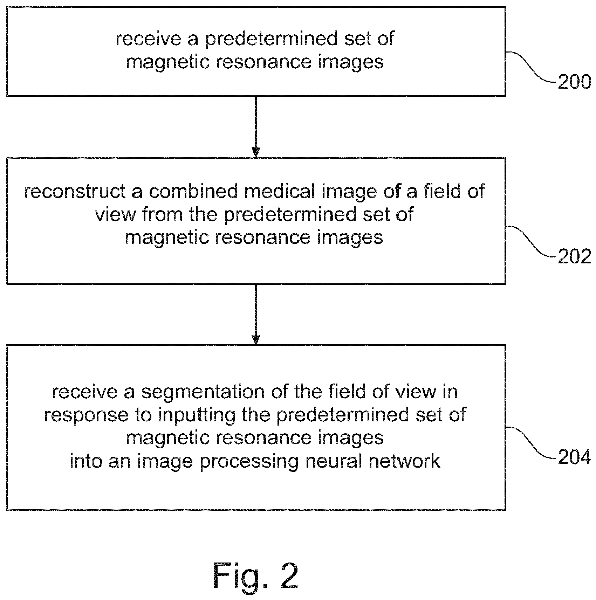

Fig. 2 shows a flowchart which illustrates a method of operating the medical system 100 of Fig. 1. First, in step 200, the predetermined set of magnetic resonance images 124 is received. Next, in step 202, the combined medical image 126 is reconstructed from the predetermined set of magnetic resonance images 124. Then, in step 204, the segmentation 128 is received in response to inputting the predetermined set of magnetic resonance images 124 into the image processing convolutional neural network 122.

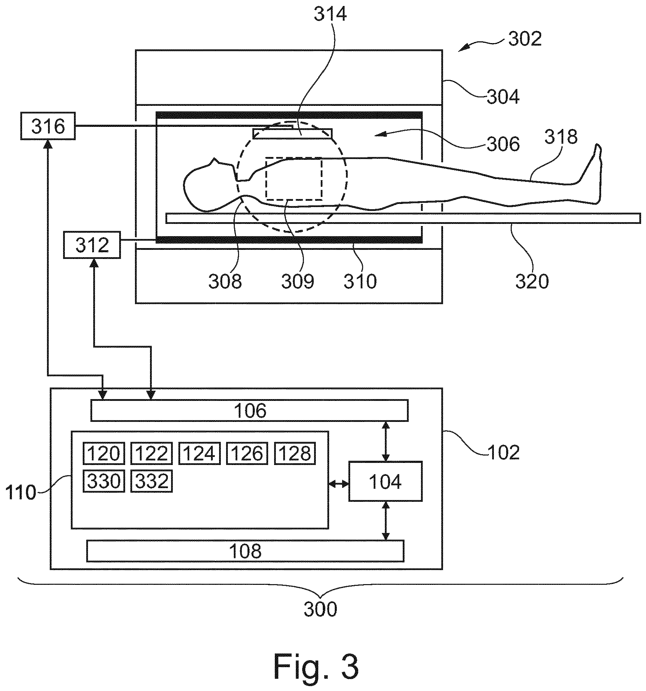

Fig. 3 illustrates a further example of the medical system 300. The medical system in Fig. 3 is similar to that as in Fig. 1 except that it additionally comprises a magnetic resonance imaging system 302 that is controlled by the computational system 104. The example illustrated in Fig. 3 is a magnetic resonance imaging system 302.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102.

The memory 110 is further shown as containing pulse sequence commands 330. The pulse sequence commands 330 are commands or data which can be converted into commands which enable the computational system 104 to control the magnetic resonance imaging system to acquire k-space data 332. The memory 110 shows the k-space data 332 that has been acquired by controlling the magnetic resonance imaging system with the pulse sequence commands 330.

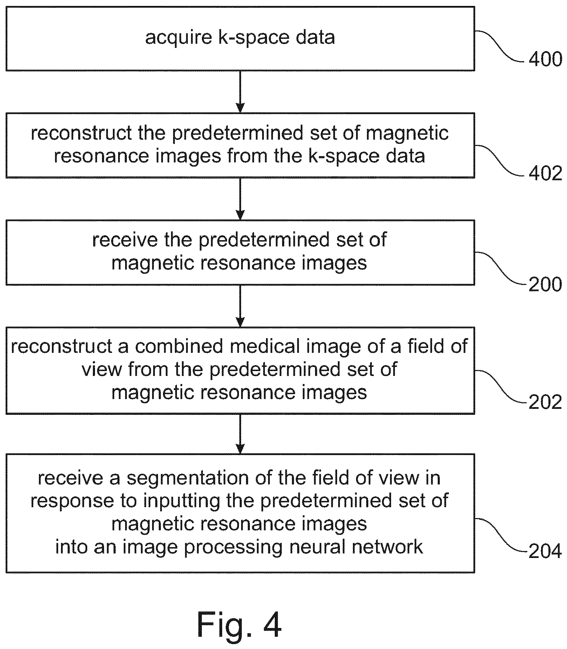

Fig. 4 shows a method of operating the medical system 300 of Fig. 3. The method starts with step 400, where the k-space data 332 is acquired by controlling the magnetic resonance imaging system 302 with the pulse sequence commands 330. Then, in step 402, the predetermined set of magnetic resonance images 124 is reconstructed from the k-space data 332. After this, the method proceeds to step 200, 202, and 204 as is illustrated in Fig. 2.

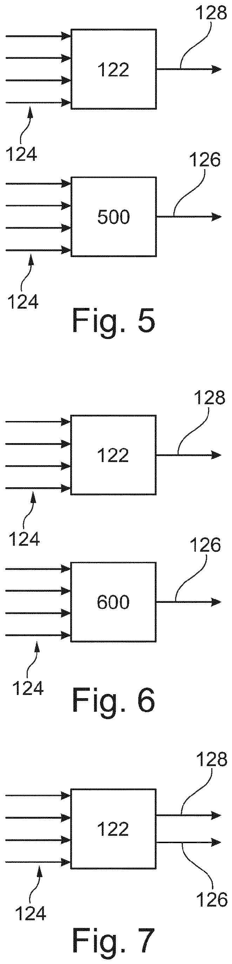

Fig. 5 illustrates one way of generating the segmentation 128 and the combined medical image 126. In this example there is a separate image processing convolutional neural network 122 and a separate image reconstruction algorithm 500. The predetermined set of magnetic resonance images 124 is input into the image processing convolutional neural network 122 and the segmentation 128 is output. The predetermined set of magnetic resonance images 124 are also input into the image reconstruction algorithm 500 and the combined medical image 126 is then output. The image reconstruction algorithm 500 could for example be implemented by the machine-executable instructions 120. In one example, the image reconstruction algorithm 500 is a magnetic resonance imaging fingerprint reconstruction algorithm.

Fig. 6 illustrates another way of obtaining the segmentation 128 and the combined medical image 126. In Fig. 6 there is a separate image processing convolutional neural network 122 and a separate reconstruction neural network 600. Again, the image processing convolutional neural network 122 takes the predetermined set of magnetic resonance images 124 as input and outputs the segmentation 128. In this example, the image reconstruction neural network 600 takes the predetermined set of magnetic resonance images 124 as input and outputs the combined medical image 126.

In Fig. 7 an alternative implementation of the image processing convolutional neural network 122 is detailed. In this example, the image processing convolutional neural network 122 again takes the predetermined set of magnetic resonance images 124 as input but then it outputs both the segmentation 128 and the combined medical image 126. A variety of neural network architectures could be used to implement the image processing convolutional neural network 122.

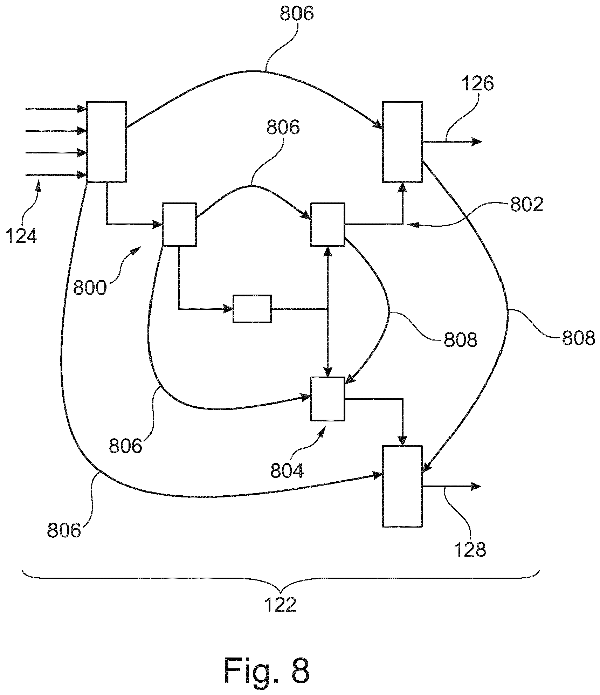

Fig. 8 illustrates one way of implementing the image processing convolutional neural network 122 as illustrated in Fig. 7. In Fig. 8 there is a so-called Y-Net which is equivalent to two U-Net architectures that have been combined. In this example, there is an encoder branch 800 which is configured for receiving the predetermined set of magnetic resonance images 124. This encoder branch 800 at its lowest node connects to a first decoder branch 802 and a second decoder branch 804. The first decoder branch 802 outputs the combined medical image 126 and the second decoder branch 804 outputs the segmentation 128. There are skip connections 806 which combine various levels of the encoder branch 800 with the first decoder branch 802 and the second decoder branch 804 respectively. There are also some optional skip connections 808. These additional skip connections 808 are skip connections between the first decoder branch 802 and the second decoder branch 804. The additional skip connections 808 may be useful because they help the system to produce a combined medical image 126 that matches well with the segmentation 128.

There is a strong interest to develop (AI) models that can detect abnormalities of (MR) images or perform segmentations to facilitate radiologists' reading efficiency or to steer the MRI scanner to suggest additional scans to be performed. The known approach takes established MR contrast-weighted images and learns the model to perform based on radiologists' ratings.

Alternatively, for fast imaging and additional confidence, approaches are taken to derive parameter maps (like T1, T2) and generate synthetic contrasts from a single MR sequence, where the individual images do not resemble known and preferred MR contrasts (Synthetic MR, MRF, MRSTAT etc.). This additional processing step may introduce bias and noise to the derived maps and images, and may not be the best input for (biomarker) classifier networks.

Examples may possibly have one or more of the following features:
1. Implementation of a neural network (U-net, cascaded U-net, or GAN, or other) to directly derive the classifier normal/abnormal from the 'raw', non-standard MR contrasts in sequences like SyntAc, QALAS, SAGE, MRF.
2. Integrate this classifier with deep learning models for image reconstruction and parameter map derivation using a so-called 'multi-tasking' architecture
   a. A specialization of this architecture is to use a single encoder branch and multiple decoder branches with skip-connections
   b. Another multi-task decoder branch would be volumetric assessment of esp. brain structures
3. Include regularization of the network by (probabilistic) matching the reconstructed images to brain templates or atlases
   a. Age specific
   b. Ethnicity specific

There is a strong variability in radiologist's preferences for acquiring anatomical and functional MR images. The only commonality, though, is that standards of practice (like ACR, for brain: https://www.acr.org/-/media/ACR/Files/Practice-Parameters/MR-Brain.pdf) prescribe review of defined contrast-weightings, such as T1, T2, T2^{∗}, FLAIR in the brain, often augmented with Diffusion and Perfusion.

Further standardization of the radiological practices is being pursued through, amongst others, initiatives to acquire datasets that can be used to derive quantitative maps of T1, T2, PD, T2^{∗} and potentially other parameters. Such methods are known as for example MRF, Synthetic MR, or SAGE. These methods perform a multi-parametric fit to derive quantitative maps, and then generate virtual or synthetic contrast images for review by the radiologist. Such fitting procedures introduce noise and bias, depending on the SNR and "parameter footprint" of the raw images. Note that these raw images are sampled at echo times, repetition times, and inversion times such that none of them represents a desired T1, T2, PD or FLAIR contrast weighting. The derived images for radiological review are an algebraic or algorithmic combination of the source images, which can further amplify the noise and bias inherent to the fitting procedure of the T1, T2, PD maps. Known standard deviation levels for the T1 and T2 fits are in the order of 6%-8%.

Standardization of image appearance is particularly important when such data is used to feed into Deep Learning models or Neural Networks for classification and/or anomaly detection. Such NNs are generally sensitive to the characteristics of the training data, such as pixel resolution but also noise color. Notably, the noise color of synthetic images will be very different from the source images, as a result of the fitting and combination steps, which are both non-linear filters. The consequence is that a network trained on annotated "routine" or "classical" MR images with annotations by radiologists is likely to fail when being fed with such synthetic images. This in addition to the fact that the synthetic images are subject to additional uncertainty in terms of contrast weighting due to the previously described fitting uncertainties. The latter is a good reason to avoid implementing a neural network trained on the parameter maps or the synthetic images, even if the classifiers and segmentations were provided by radiologists on those images.

To circumvent these problems, this invention teaches to train the neural network on the native or "atypical" MR images (with non-radiological appearance) prior to feeding them into the parameter fitting or image synthesis tasks. Segmentation and classification of lesions still needs to be performed by the Radiologist based on the "classical" MR images per his/her preference, and the annotations are then transferred onto the "atypical" images. Note that this relates to a set of 8 or more different contrasts, and the network will learn its classification for each of the contrasts. Using an approach like "multi-tasking regularization" all classification tasks are linked together for a more robust neural network.

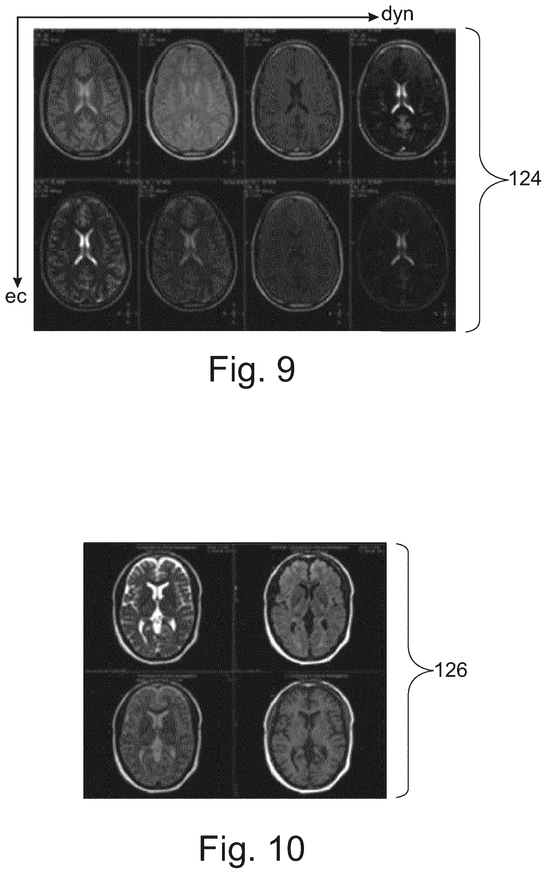

An example of such "atypical" images is illustrated in Fig. 9. Fig. 9 shows a number of magnetic resonance images that have been acquired using the SyntAC technique. These are a predetermined set of magnetic resonance images 124. These images can then be used to create a number of synthetic magnetic resonance images having different weighting modalities.

Fig. 10 illustrates a number of combined medical images 126 that have been reconstructed using the predetermined set of magnetic resonance images 124 from Fig. 9. These are synthetic magnetic resonance images 126.

Similar examples can be considered for MRF or MRSTAT like acquisitions, where the source images are subsampled reconstructions.

Examples may use images as input for the classification, and not MR raw data, since the raw data contains all spatial information at all sample points. The vast dynamic range of k-space, and the inherent spatial mixing, makes it more difficult for a well-performing neural network to perform classification. Performing a Fourier transform is a linear process and does not lead to loss of generality of the classification approach. Learning the k-space to 'segmentation' or 'classification' in image space involves many neural network layers for such an arbitrary and trivial task as performing the FFT, and end-to-end NNs for image reconstruction are known to poorly generalize.

The training approach of the neural network, as described, involves e.g.
- Acquire the regular radiological images
- Acquire the "atypical images"
- Radiological review and label generation on the radiological images
- Transfer the label to the atypical images
- Train the network (based on an appropriate selection of test, tune and validation data, and known approaches like k-fold cross-training and leave-out validation)

During deployment, the network is fed by the "atypical images" and generates pixel classes such as "normal WM, normal GM, CSF, Bone, unclassifiable, ...".

In addition, concepts like Conformal Prediction can be used to represent these classes and the confidence / likelihood of correctness of classification cq likelihood that the set of proposed classes contains a correct suggestion.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: image processing convolutional neural network
- 124: predetermined set of magnetic resonance images
- 126: combined medical image
- 128: segmentation
- 200: receive the predetermined set of magnetic resonance images
- 202: reconstruct a combined medical image of the field of view from the predetermined set of magnetic resonance images
- 204: receive the segmentation in response to inputting the predetermined set of magnetic resonance images into the image processing convolutional neural network
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: pulse sequence commands
- 332: k-space data
- 400: acquire the k-space data by controlling the magnetic resonance imaging system
- 402: reconstruct the predetermined set of magnetic resonance images from the k-space data
- 500: image reconstruction algorithm
- 600: image reconstruction neural network
- 800: encoder branch
- 802: first decoder branch
- 804: second decoder branch
- 806: skip connections
- 808: additional skip connections

## Claims

1. A medical system (100, 300) comprising:
- a memory (110) storing machine executable instructions (120) and an image processing convolutional neural network (122), wherein the image processing convolutional neural network is configured to output a segmentation (128) of a field of view (309) of a subject (318) in response to inputting a predetermined set of magnetic resonance images (124) according to a magnetic resonance imaging protocol into the image processing convolutional neural network, wherein each of the predetermined set of magnetic resonance images is descriptive of the field of view of the subject; and
- a computational system (104), wherein execution of the machine executable instructions causes the computational system to:
- receive (200) the predetermined set of magnetic resonance images;
- reconstruct (202) a combined medical image (126) of the field of view from the predetermined set of magnetic resonance images, wherein the combined medical image is a non-linear combination of the magnetic resonance images; and
- receive (204) the segmentation in response to inputting the predetermined set of magnetic resonance images into the image processing convolutional neural network.

2. . The medical system of claim 1, wherein the machine executable instructions are configured such that computational system is able to reconstruct the combined medical image when the predetermined set of magnetic resonance images have a predetermined distribution of magnetic resonance image weightings or magnetic resonance imaging contrast types.

3. . The medical system of claim 1 or 2, wherein the segmentation is a segmentation of the combined medical image.

4. . The medical system of claim 1, 2, or 3, wherein each of the predetermined set of magnetic resonance images is a non-clinical magnetic resonance image or non-canonical magnetic resonance image.

5. . The medical system of any one of the preceding claims, wherein the memory further comprises an image reconstruction neural network (600) configured to output the combined medical image in response to receiving the predetermined set of magnetic resonance images as input, wherein combined medical image is reconstructed by inputting the predetermined set of magnetic resonance images into the image reconstruction neural network.

6. . The medical system of any one of the preceding claims 3, wherein the image processing convolutional neural network is further configured for outputting the combined medical image in response to receiving the predetermined set of magnetic resonance images as input, wherein combined medical image is reconstructed by inputting the predetermined set of magnetic resonance images into the image processing convolutional neural network.

7. . The medical system of claim 6, wherein the image processing convolutional neural network comprises a single encoder branch (800) configured for receiving the predetermined set of magnetic resonance images as input, wherein the image processing convolutional neural network further comprises a first decoder branch (802) and a second decoder branch (804) each connected to the single encoder branch, wherein the first decoder branch is configured to output the combined medical image, wherein the second decoder branch is configured to output the segmentation of the combined medical image.

8. . The medical system of claim 7, wherein the image processing convolutional neural network further comprises multiple skip connections (808) between the first decoder branch and the second decoder branch.

9. . The medical system of claim 1, 2, or 3, wherein the machine executable instructions are configured to reconstruct the combined medical image algorithmically.

10. . The medical system of any one of the preceding claims, wherein the magnetic resonance imaging protocol is any one of the following: a magnetic resonance fingerprinting protocol, a SyntAc protocol, a QRAPMASTER protocol, a QALAS protocol, a MaGIC protocol, a MP2RAGEME protocol, a EPImix protocol, a DESS magnetic resonance imaging protocol, a TESS magnetic resonance imaging protocol, an ultrashort echo time magnetic resonance imaging protocol, a DIXON magnetic resonance imaging protocol, a SAGE magnetic resonance imaging protocol, a QuICS magnetic resonance imaging protocol, and an MRSTAT magnetic resonance imaging protocol.

11. . The medical system of any one of the preceding claims, wherein the combined medical image is any one of the following: a synthetic magnetic resonance image, a synthetic medical image, a synthetic proton density weighted magnetic resonance image, a synthetic T1 weighted magnetic resonance image, a synthetic T2 weighted magnetic resonance image, a synthetic T2-star weighted magnetic resonance image, a synthetic FLAIR magnetic resonance image, a quantitative map, a pseudo computed tomography image, and a pseudo X-ray image.

12. . The medical system of any one of the preceding claims, wherein the medical system further comprises a magnetic resonance imaging system (302), wherein the memory further contains pulse sequence commands (330) configured to control the magnetic resonance imaging system to acquire k-space data according to the magnetic resonance imaging protocol, wherein execution of the machine executable instructions further causes the computational system to:
- acquire (400) the k-space data by controlling the magnetic resonance imaging system; and
- reconstruct (402) the predetermined set of magnetic resonance images from the k-space data.

13. . The medical system of any one of the preceding claims, wherein the segmentation of the combined medical image comprises any one of the following: an anatomical segmentation, an identification of a damaged tissue region, an identification of an anatomical abnormality region, an identification of a lesion region, and combinations thereof.

14. . A computer program comprising machine executable instructions (120) and an image processing convolutional neural network (122), wherein the image processing convolutional neural network is configured to output a segmentation (128) of a field of view (309) of a subject in response to inputting a predetermined set of magnetic resonance images (124) according to a magnetic resonance imaging protocol into the image processing convolutional neural network, wherein each of the predetermined set of magnetic resonance images is descriptive of the field of view of the subject, wherein execution of the machine executable instructions causes a computational system to:
- receive (200) the predetermined set of magnetic resonance images;
- reconstruct (202) a combined medical image (126) of the field of view from the predetermined set of magnetic resonance images, wherein the combined medical image is a non-linear combination of the magnetic resonance images; and
- receive (204) the segmentation in response to inputting the predetermined set of magnetic resonance images into the image processing convolutional neural network.

15. . A method of medical imaging, wherein the method comprises:
- receiving (200) a predetermined set of magnetic resonance images (124), wherein each of the predetermined set of magnetic resonance images is descriptive of a field of view (309) of a subject (318);
- reconstructing (202) a combined medical image (126) of the field of view from the predetermined set of magnetic resonance images, wherein the combined medical image is a non-linear combination of the magnetic resonance images; and
- receiving (204) a segmentation (128) in response to inputting the predetermined set of magnetic resonance images into an image processing convolutional neural network, wherein the image processing convolutional neural network is configured to output the segmentation of the field of view of the subject in response to inputting the predetermined set of magnetic resonance images according to a magnetic resonance imaging protocol into the image processing convolutional neural network.
